# EUROPEAN PATENT APPLICATION

(11) **EP 4 501 214 A1**
(43) Date of publication of application: **05.02.2025**
(21) Application number: 23778813.8
(22) Date of filing: 30.01.2023
(51) Int. Cl.: A61B 5/00

(54) **INFORMATION PROCESSING DEVICE, METHOD FOR OPERATING INFORMATION PROCESSING DEVICE, AND PROGRAM FOR OPERATING INFORMATION PROCESSING DEVICE**

(30) Priority: 29.03.2022 JP 2022054510
(71) Applicant: FUJIFILM Corporation, Tokyo 106-8620 (JP)
(72) Inventor: TOMINAGA, Shunsuke, Ashigarakami-gun, Kanagawa 258-8577 (JP)
(74) Representative: HGF
(86) International application number: PCT/JP2023/002931
(87) International publication number: WO 2023/188776

(57) **Abstract**

An information processing apparatus includes a processor configured to: acquire an organ image obtained by imaging an organ of a subject; detect an abnormal portion, in which an abnormality is estimated to occur, from the organ image; register the organ image with a reference image having positional information of a plurality of anatomical parts of the organ to define the anatomical parts in the organ image; and output degree information indicating a degree of occurrence of the abnormal portion in each of the plurality of anatomical parts.

## Description

### BACKGROUND OF THE INVENTION

### 1. Field of the Invention

The technology of the present disclosure relates to an information processing apparatus, a method for operating an information processing apparatus, and a program for operating an information processing apparatus.

### 2. Description of the Related Art

In a medical field, various evaluations based on an organ image obtained by imaging organs of a subject are performed. For example, in a test that administers a candidate substance for a drug to a subject, such as a rat, and evaluates the efficacy and toxicity of the candidate substance, a section image obtained by imaging a tissue section (a brain section, a heart section, or the like) collected by autopsying the subject is used as the organ image. In the related art, a pathologist or the like observes the organ image to perform the evaluation. However, with the recent progress of an image analysis technique, a technique has been developed that automatically performs the evaluation without causing the expert trouble. For example, JP2020-533725A discloses a technique that applies a machine learning model to a plurality of patch images obtained by subdividing a section image and determines whether or not an abnormality, such as cancer, has occurred in a tissue section included in the patch image.

### SUMMARY OF THE INVENTION

Some organs have a plurality of anatomical parts. For example, the brain has anatomical parts such as a cerebral cortex, a hippocampal formation, a thalamus, and an amygdala. In the evaluation using the organ image, there is a demand for ascertaining how much the abnormality has occurred in each of the anatomical parts. However, in the technique disclosed in JP2020-533725A, it is possible to ascertain whether or not an abnormality occurs in the tissue section included in the patch image, but it is difficult to ascertain how much the abnormality occurs in each of the anatomical parts.

An embodiment according to the technology of the present disclosure provides an information processing apparatus, a method for operating an information processing apparatus, and a program for operating an information processing apparatus that can easily ascertain how much an abnormality occurs in each of anatomical parts of an organ.

According to the present disclosure, there is provided an information processing apparatus comprising a processor configured to: acquire an organ image obtained by imaging an organ of a subject; detect an abnormal portion, in which an abnormality is estimated to occur, from the organ image; register the organ image with a reference image having positional information of a plurality of anatomical parts of the organ to define the anatomical parts in the organ image; and output degree information indicating a degree of occurrence of the abnormal portion in each of the plurality of anatomical parts.

Preferably, the processor is configured to: extract a feature amount of a region of interest in the organ image; and determine whether or not the region of interest is the abnormal portion on the basis of a difference between the feature amount extracted from the region of interest and a reference feature amount.

Preferably, the reference feature amount is a feature amount extracted from a region around the region of interest.

Preferably, the organ image is an image used in an evaluation test for evaluating a candidate substance for a drug, and the reference feature amount is a feature amount extracted from an organ image of a subject to which the candidate substance has not been administered.

Preferably, the processor is configured to perform control to display the defined anatomical part on a display to be superimposed on the organ image.

Preferably, the processor is configured to receive an instruction to correct the defined anatomical part.

Preferably, the processor is configured to output the degree information generated on the basis of the anatomical part corrected according to the correction instruction.

Preferably, the degree information includes the number of the abnormal portions in each of the plurality of anatomical parts and an area ratio of the abnormal portion in each of the plurality of anatomical parts.

Preferably, the organ image is a section image obtained by imaging a tissue section of the organ.

According to another aspect of the present disclosure, there is provided a method for operating an information processing apparatus. The method comprises: acquiring an organ image obtained by imaging an organ of a subject; detecting an abnormal portion, in which an abnormality is estimated to occur, from the organ image; registering the organ image with a reference image having positional information of a plurality of anatomical parts of the organ to define the anatomical parts in the organ image; and outputting degree information indicating a degree of occurrence of the abnormal portion in each of the plurality of anatomical parts.

According to another aspect of the present disclosure, there is provided a program for operating an information processing apparatus. The program causes a computer to execute a process comprising: acquiring an organ image obtained by imaging an organ of a subject; detecting an abnormal portion, in which an abnormality is estimated to occur, from the organ image; registering the organ image with a reference image having positional information of a plurality of anatomical parts of the organ to define the anatomical parts in the organ image; and outputting degree information indicating a degree of occurrence of the abnormal portion in each of the plurality of anatomical parts.

According to the technology of the present disclosure, it is possible to provide an information processing apparatus, a method for operating an information processing apparatus, and a program for operating an information processing apparatus that can easily ascertain how much an abnormality occurs in each of anatomical parts of an organ.

### BRIEF DESCRIPTION OF THE DRAWINGS

Fig. 1 is a diagram illustrating steps of an evaluation test, section images, and a drug discovery support apparatus.
Fig. 2 is a diagram illustrating an administration group and a control group.
Fig. 3 is a block diagram illustrating a computer constituting the drug discovery support apparatus.
Fig. 4 is a block diagram illustrating processing units of a CPU of the drug discovery support apparatus.
Fig. 5 is a diagram illustrating patch images obtained by subdividing a section image.
Fig. 6 is a diagram illustrating an aspect in which a feature amount extractor extracts a feature amount from the patch image.
Fig. 7 is a diagram illustrating a structure of the feature amount extractor.
Fig. 8 is a diagram illustrating a process in a learning phase of an autoencoder.
Fig. 9 is a diagram illustrating a process of a detection unit.
Fig. 10 is a diagram illustrating a detection result.
Fig. 11 is a diagram illustrating a reference image.
Fig. 12 is a diagram illustrating a process of a definition unit.
Fig. 13 is a diagram illustrating a definition result.
Fig. 14 is a diagram illustrating degree information.
Fig. 15 is a diagram illustrating a drug discovery support screen on which the section images are displayed.
Fig. 16 is a diagram illustrating a drug discovery support screen on which the degree information is displayed.
Fig. 17 is a flowchart illustrating a processing procedure of the drug discovery support apparatus.
Fig. 18 is a diagram illustrating another example of the degree information displayed on the drug discovery support screen.
Fig. 19 is a diagram illustrating still another example of the degree information displayed on the drug discovery support screen.
Fig. 20 is a diagram illustrating a process of a detection unit according to a second embodiment.
Fig. 21 is a diagram illustrating a drug discovery support screen on which a defined anatomical part is displayed to be superimposed on the section image.
Fig. 22 is a diagram illustrating a drug discovery support screen for receiving an instruction to correct the anatomical part.
Fig. 23 is a diagram illustrating a 3_2-th embodiment in which the degree information is generated on the basis of the anatomical part corrected according to the correction instruction.

### DESCRIPTION OF THE PREFERRED EMBODIMENTS

### [First Embodiment]

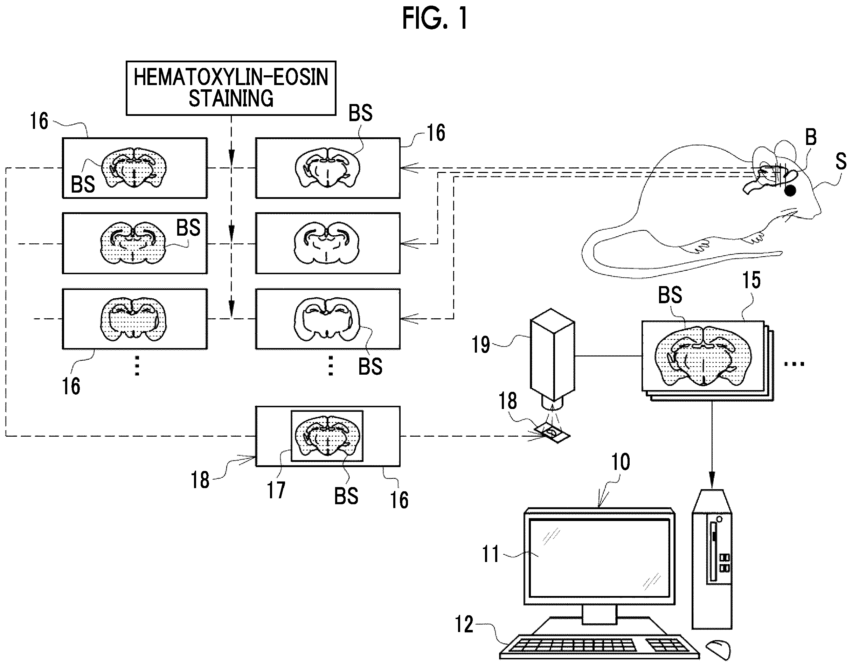

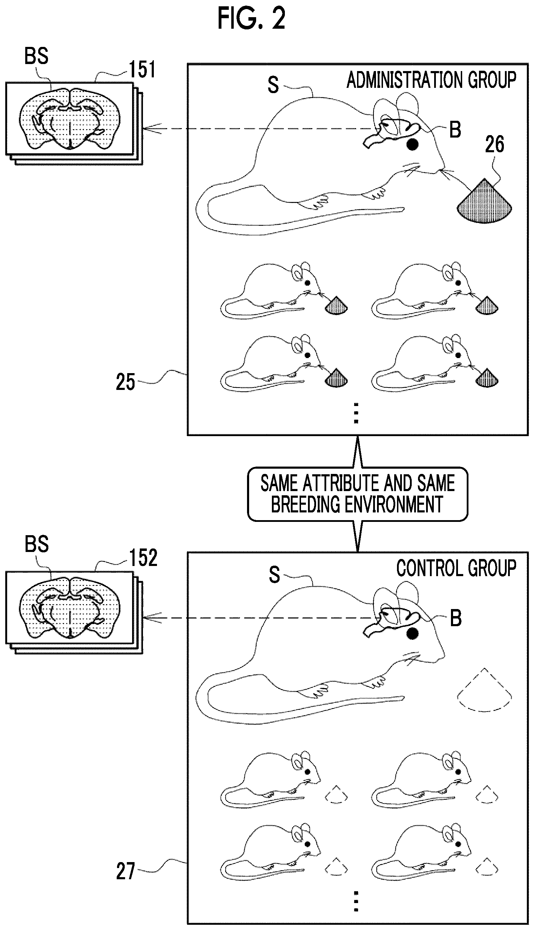

For example, as illustrated in Fig. 1, a drug discovery support apparatus 10 is an example of an "information processing apparatus" according to the technology of the present disclosure and is used to evaluate the efficacy and toxicity of a candidate substance 26 (see Fig. 2) for a drug. The drug discovery support apparatus 10 is, for example, a desktop personal computer and comprises a display 11 that displays various screens and an input device 12 that consists of a keyboard, a mouse, a touch panel, and/or a microphone for voice input. The drug discovery support apparatus 10 is operated by an operator such as a staff member of a pharmaceutical facility involved in drug development. In addition, the display 11 is an example of a "display" according to the technology of the present disclosure.

A section image 15 is input to the drug discovery support apparatus 10. The section image 15 is generated, for example, by the following procedure. First, a subject S, such as a rat, that is prepared to evaluate the candidate substance 26 is autopsied, and a plurality of tissue sections (hereinafter, referred to as brain sections) BS of coronal sections of an organ, here, a brain B of the subject S are collected. Then, the collected brain sections BS are attached one by one to a slide glass 16. Then, the brain sections BS are stained, here, with a hematoxylin-eosin dye. Then, the stained brain sections BS are covered with a cover glass 17 to complete a slide specimen 18. Then, the slide specimen 18 is set in an imaging device 19, such as a digital optical microscope, and the section image 15 is captured by the imaging device 19. The section image 15 obtained in this way is given subject identification data (ID) for uniquely identifying the subject S, an image ID for uniquely identifying the section image 15, a slice position of the coronal section, an imaging date and time, and the like. The section image 15 is an example of an "organ image" and an "image used in an evaluation test for evaluating a candidate substance for a drug" according to the technology of the present disclosure. In addition, the staining may be staining with a hematoxylin dye alone, staining with a nuclear fast red dye, or the like.

For example, as illustrated in Fig. 2, the section images 15 input to the drug discovery support apparatus 10 include a first section image 151 and a second section image 152. The first section image 151 is an image obtained by imaging the brain section BS of the subject S in an administration group 25. The administration group 25 is composed of a plurality of subjects S to which the candidate substance 26 for a drug has been administered. The number of subjects S constituting the administration group 25 is, for example, about 5 to 10. Since a plurality of first section images 151 are obtained from one subject S, the number of first section images 151 obtained from the administration group 25 is a value obtained by multiplying the number of first section images 151 obtained from one subject S by the number of subjects S. For example, in a case where the number of first section images 151 obtained from one subject S is 10 and the number of subjects S constituting the administration group 25 is 10, 100 (= 10 × 10) first section images 151 are obtained.

The second section image 152 is an image obtained by imaging the brain section BS of the subject S in a control group 27. The control group 27 is composed of a plurality of subjects S to which the candidate substance 26 for a drug has not been administered, contrary to the administration group 25. The subjects S constituting the control group 27 have the same attributes and are placed under the same breeding environment as the subjects S constituting the administration group 25. The same attributes include, for example, the same weekly age and/or the same gender. In addition, the same attributes also include the same weekly age composition ratio and/or the same gender composition ratio (for example, five males and five females). The same breeding environment means, for example, that feed is the same, that the temperature and humidity of a breeding space are the same, and/or that the size of the breeding space is the same. The "same" in the same breeding environment indicates not only the exact same, but also the same including an error that is generally allowed in the technical field to which the technology of the present disclosure belongs and that does not go against the gist of the technology of the present disclosure. The number of subjects constituting the control group 27 is also, for example, about 5 to 10 that is equal to the number of subjects S in the administration group 25. Similarly to the first section images 151, a plurality of second section images 152 are obtained from one subject S. Therefore, the number of second section images 152 obtained from the control group 27 is a value obtained by multiplying the number of second section images 152 obtained from one subject S by the number of subjects S. In the following description, the first section image 151 and the second section image 152 are collectively referred to as section images 15 in a case where the first section image 151 and the second section image 152 do not need to be particularly distinguished from each other.

In addition, a plurality of groups having different doses of the candidate substance 26 are present in the administration group 25. For example, the dose of the candidate substance 26 varies in three stages of a high-dose group, a medium-dose group, and a low-dose group. This makes it possible to determine the influence of the dose of the candidate substance 26 on the subject S.

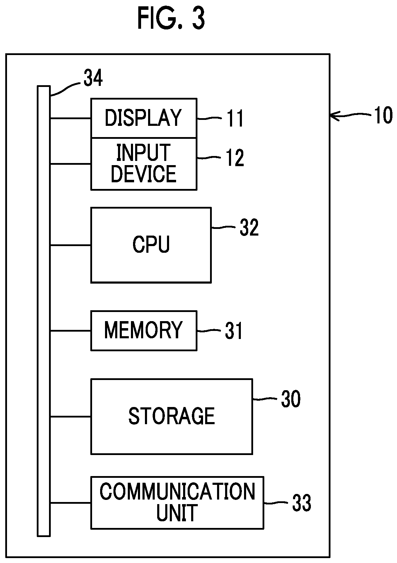

For example, as illustrated in Fig. 3, a computer constituting the drug discovery support apparatus 10 comprises a storage 30, a memory 31, a central processing unit (CPU) 32, and a communication unit 33 in addition to the display 11 and the input device 12. These components are connected to one another via a bus line 34.

The storage 30 is a hard disk drive that is provided in the computer constituting the drug discovery support apparatus 10 or that is connected via a cable or a network. Alternatively, the storage 30 is a disk array in which a plurality of hard disk drives are connected in series. The storage 30 stores a control program, such as an operating system, various application programs, various types of data associated with these programs, and the like. In addition, a solid state drive may be used instead of the hard disk drive.

The memory 31 is a work memory for the CPU 32 to execute processes. The CPU 32 loads the program stored in the storage 30 into the memory 31 and executes a process corresponding to the program. Therefore, the CPU 32 controls the overall operation of each unit of the computer. In addition, the CPU 32 is an example of a "processor" according to the technology of the present disclosure. Further, the memory 31 may be provided in the CPU 32. The communication unit 33 controls the transmission of various types of information to an external device such as the imaging device 19.

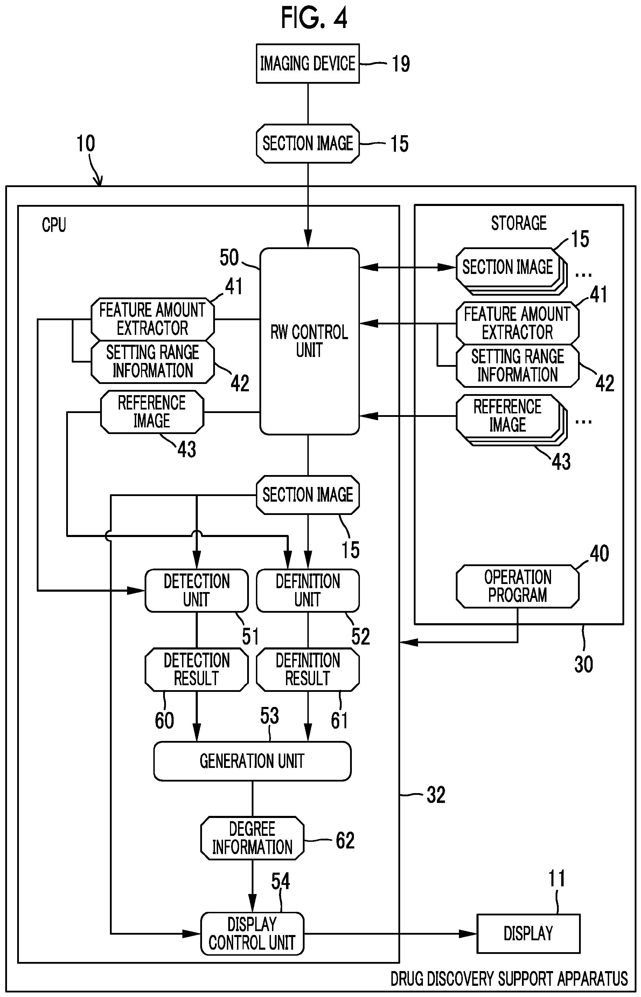

For example, as illustrated in Fig. 4, an operation program 40 is stored in the storage 30 of the drug discovery support apparatus 10. The operation program 40 is an application program for causing the computer to function as the drug discovery support apparatus 10. That is, the operation program 40 is an example of a "program for operating an information processing apparatus" according to the technology of the present disclosure. A feature amount extractor 41, setting range information 42, a reference image 43, and the like are also stored in the storage 30.

In a case where the operation program 40 is started, the CPU 32 of the computer constituting the drug discovery support apparatus 10 functions as a read write (hereinafter, abbreviated to RW) control unit 50, a detection unit 51, a definition unit 52, a generation unit 53, and a display control unit 54 in cooperation with the memory 31 and the like.

The RW control unit 50 controls the storage of various types of data in the storage 30 and the reading-out of various types of data in the storage 30. For example, the RW control unit 50 stores the section image 15 from the imaging device 19 in the storage 30.

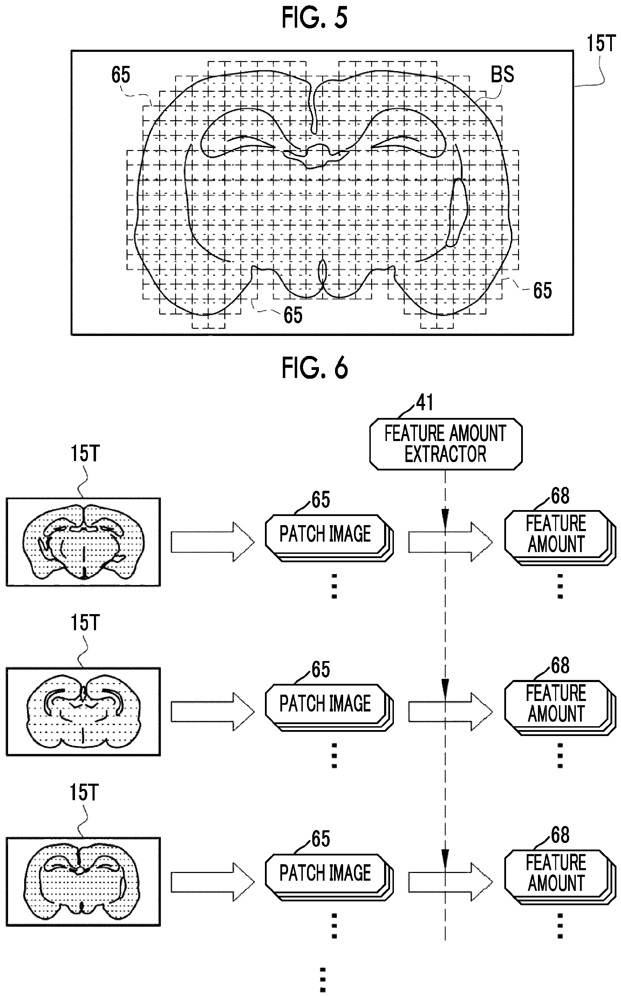

The RW control unit 50 reads out the section image 15 from the storage 30 to acquire the section image 15. The RW control unit 50 outputs the section image 15 to the detection unit 51, the definition unit 52, and the display control unit 54. The section image 15 output from the RW control unit 50 to the detection unit 51 and the like is an object in which the degree of occurrence of an abnormal portion of the brain section BS is to be ascertained. Hereinafter, the section image 15 in which the degree of occurrence of the abnormal portion of the brain section BS is to be ascertained is referred to as a target section image 15T (see Fig. 5 and the like). The target section image 15T is, for example, all of a plurality of section images 15 obtained from one subject S among a plurality of subjects S constituting the administration group 25.

The RW control unit 50 reads out the feature amount extractor 41 and the setting range information 42 from the storage 30 and outputs the feature amount extractor 41 and the setting range information 42 to the detection unit 51. In addition, the RW control unit 50 reads out the reference image 43 from the storage 30 and outputs the reference image 43 to the definition unit 52.

The detection unit 51 detects an abnormal portion, in which an abnormality is estimated to occur, from the target section image 15T using the feature amount extractor 41. The abnormal portion is a portion in which a lesion, such as a tumor, inflammation, a cyst, or an infiltrate, that is not seen in the normal brain section BS has occurred. The detection unit 51 outputs a detection result 60 of the abnormal portion to the generation unit 53.

The definition unit 52 registers the target section image 15T with the reference image 43 to define a plurality of anatomical parts of the brain B in the target section image 15T. The definition unit 52 outputs a definition result 61 of the anatomical parts of the brain B to the generation unit 53. In addition, the definition unit 52 performs a process of defining an anatomical part in parallel with the process of detecting the abnormal portion using the detection unit 51.

The generation unit 53 generates degree information 62 indicating the degree of occurrence of an abnormal portion in each of the plurality of anatomical parts of the brain B on the basis of the detection result 60 and the definition result 61. The generation unit 53 outputs the degree information 62 to the display control unit 54.

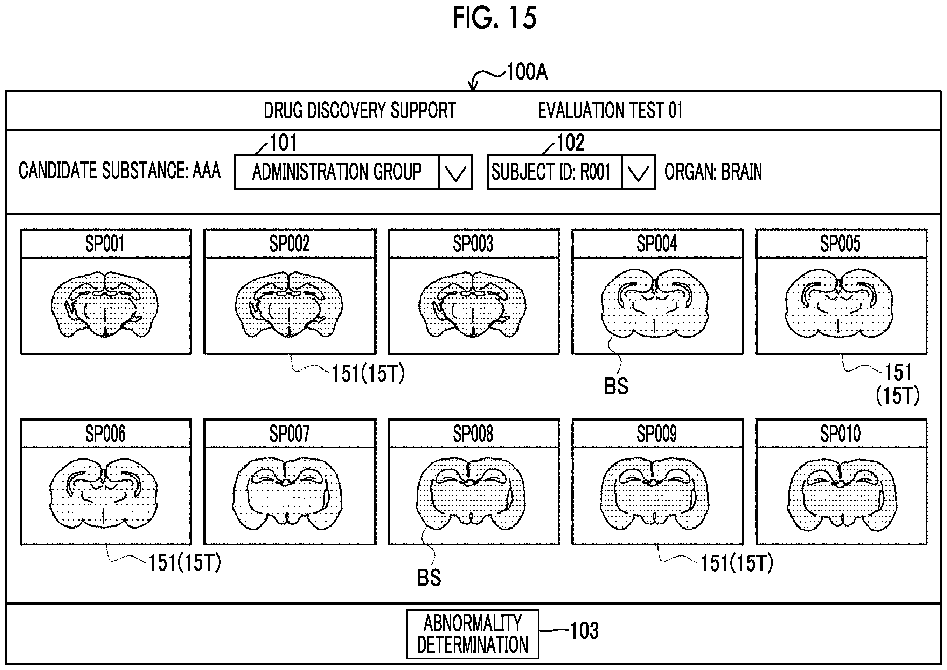

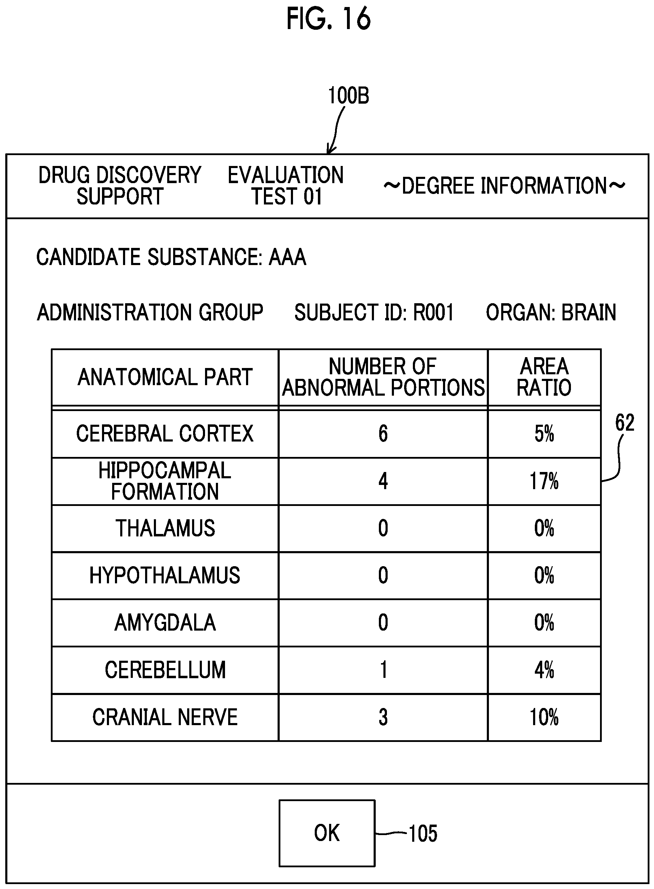

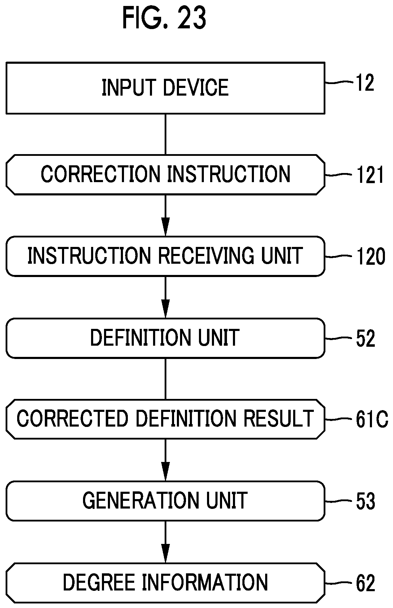

The display control unit 54 performs control to display various screens on the display 11. The various screens include, for example, a drug discovery support screen 100A (see Fig. 15) on which the section image 15 is displayed and a drug discovery support screen 100B (see Fig. 16) on which the degree information 62 is displayed. Further, in addition to these processing units 50 to 54, an instruction receiving unit 120 (see Fig. 23) that receives various operation instructions from the input device 12 is constructed in the CPU 32.

For example, as illustrated in Fig. 5, the detection unit 51 recognizes the brain section BS included in the target section image 15T using a known image recognition technique and subdivides the recognized brain section BS into a plurality of patch images 65. The patch image 65 has a preset size that can be handled by the feature amount extractor 41. Further, in Fig. 5, the patch image 65 does not have a region that overlaps other patch images 65. However, the patch image 65 may partially overlap other patch images 65.

For example, as illustrated in Fig. 6, the detection unit 51 extracts a feature amount 68 for each of the plurality of patch images 65 subdivided from the target section image 15T using the feature amount extractor 41.

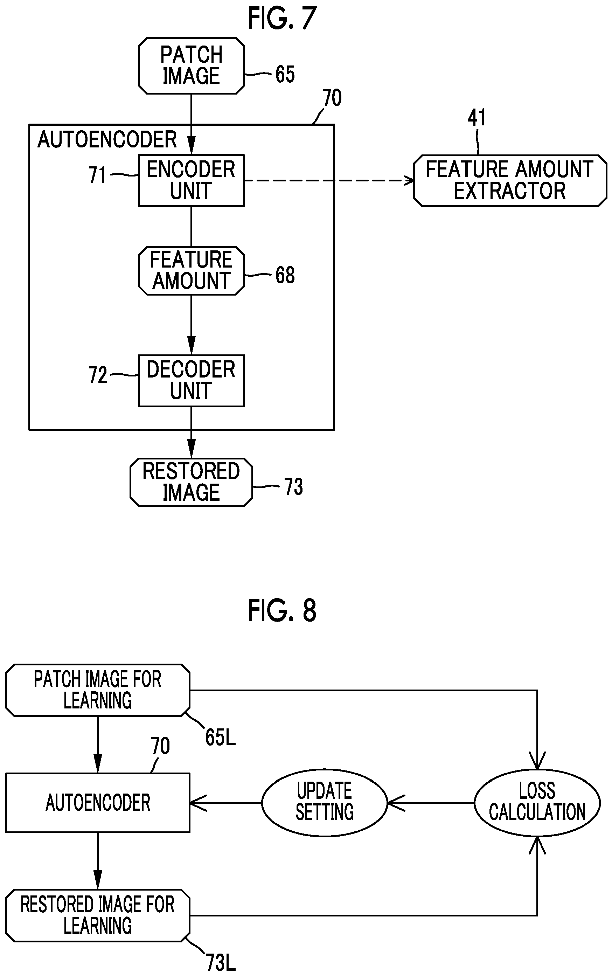

For example, as illustrated in Fig. 7, an encoder unit 71 of an autoencoder 70 is used as the feature amount extractor 41. The autoencoder 70 includes a decoder unit 72 in addition to the encoder unit 71. The patch image 65 is input to the encoder unit 71. The encoder unit 71 converts the patch image 65 into the feature amount 68. The encoder unit 71 transmits the feature amount 68 to the decoder unit 72. The decoder unit 72 generates a restored image 73 of the patch image 65 from the feature amount 68.

As is well known, the encoder unit 71 includes a convolutional layer that performs a convolution process using a filter, a pooling layer that performs a pooling process, such as a maximum pooling process, and the like. The same applies to the decoder unit 72. The encoder unit 71 repeatedly performs the convolution process using the convolutional layer and the pooling process using the pooling layer on the input patch image 65 a plurality of times to extract the feature amount 68. The extracted feature amount 68 indicates the shape and texture features of the brain section BS included in the patch image 65. The feature amount 68 is a set of a plurality of numerical values. That is, the feature amount 68 is multi-dimensional data. The number of dimensions of the feature amount 68 is, for example, 512, 1024, 2048, or the like.

For example, as illustrated in Fig. 8, in a learning phase before the encoder unit 71 is used as the feature amount extractor 41, a patch image 65L for learning is input to the autoencoder 70 to train the autoencoder 70. The autoencoder 70 outputs a restored image 73L for learning for the patch image 65L for learning. The loss calculation of the autoencoder 70 using a loss function is performed on the basis of the patch image 65L for learning and the restored image 73L for learning. Then, the update of various coefficients (for example, coefficients of convolutional layer filters) of the autoencoder 70 is set according to the results of the loss calculation, and the autoencoder 70 is updated according to the update setting. Here, the patch image 65L for learning is supplied from a plurality of patch images 65 obtained by subdividing the brain section BS included in the section image 15 obtained in the evaluation test of the candidate substance for the drug developed in the past.

In the learning phase of the autoencoder 70, the series of processes of the input of the patch image 65L for learning to the autoencoder 70, the output of the restored image 73L for learning from the autoencoder 70, the loss calculation, the update setting, and the update of the autoencoder 70 is repeatedly performed while the patch image 65L for learning is exchanged. The repetition of the series of processes is ended in a case where the accuracy of restoration from the patch image 65L for learning to the restored image 73L for learning reaches a predetermined setting level. The encoder unit 71 of the autoencoder 70 whose restoration accuracy has reached the setting level in this way is stored as the feature amount extractor 41 in the storage 30 of the drug discovery support apparatus 10. In addition, in a case where the series of processes is repeated a set number of times, the training may be ended, regardless of the accuracy of restoration from the patch image 65L for learning to the restored image 73L for learning.

This training of the autoencoder 70 may be performed by the drug discovery support apparatus 10 or may be performed by an apparatus different from the drug discovery support apparatus 10. In the latter case, the feature amount extractor 41 is transmitted from another apparatus to the drug discovery support apparatus 10, and the RW control unit 50 stores the feature amount extractor 41 in the storage 30.

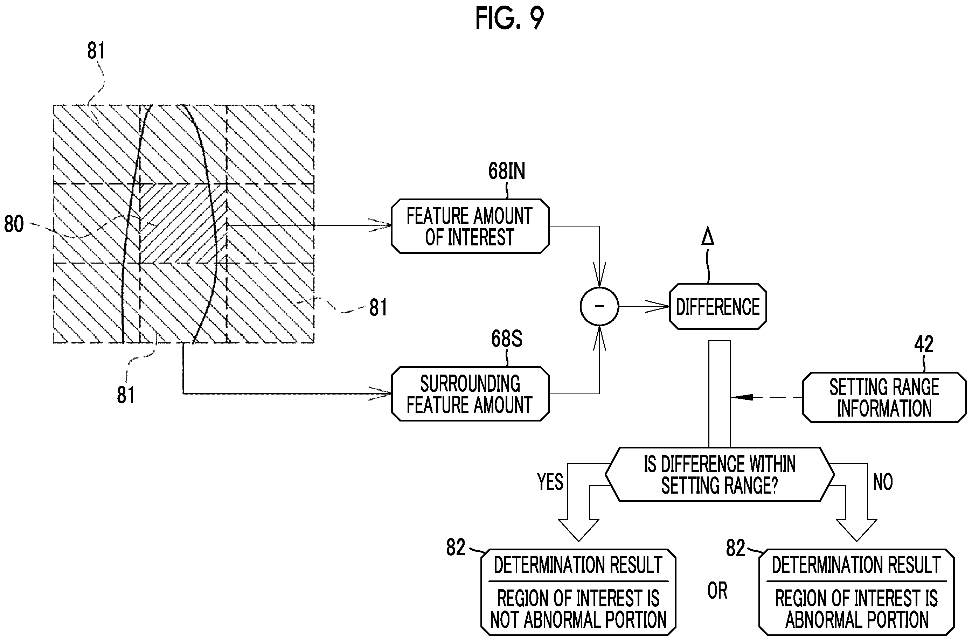

For example, as illustrated in Fig. 9, the detection unit 51 determines whether or not a region of interest 80 is an abnormal portion on the basis of a difference Δ between a feature amount of interest 68IN extracted from the region of interest 80 and a surrounding feature amount 68S extracted from a surrounding region 81. The region of interest 80 is a region to be determined whether or not it is the abnormal portion. The region of interest 80 is, for example, a region of one or a plurality of (four, nine, 16, or the like) patch images 65. The surrounding region 81 is a region around the region of interest 80. The detection unit 51 performs the determination each time the region of interest 80 is moved one by one. In addition, the detection unit 51 changes the size of the region of interest 80 (the number of patch images 65 constituting the region of interest 80) in some stages to perform the determination. In Fig. 9, the number of surrounding regions 81 are eight. However, in a case where the region of interest 80 is located near the end of the brain section BS, the number of surrounding regions 81 is less than eight. The surrounding feature amount 68S is a representative value, for example, an average value of the feature amounts 68 extracted from a plurality of surrounding regions 81. The surrounding feature amount 68S is an example of a "reference feature amount" according to the technology of the present disclosure.

The detection unit 51 compares the difference Δ between the feature amount of interest 68IN and the surrounding feature amount 68S with the setting range of the setting range information 42. The setting range information 42 includes a lower limit value and an upper limit value indicating the setting range. In a case where the difference Δ is within the setting range (the lower limit value ≤ the difference Δ ≤ the upper limit value), the detection unit 51 outputs a determination result 82 indicating that the region of interest 80 is not the abnormal portion. On the other hand, in a case where the difference Δ is out of the setting range (the difference Δ < the lower limit value, or the difference Δ > the upper limit value), the detection unit 51 outputs a determination result 82 indicating that the region of interest 80 is the abnormal portion.

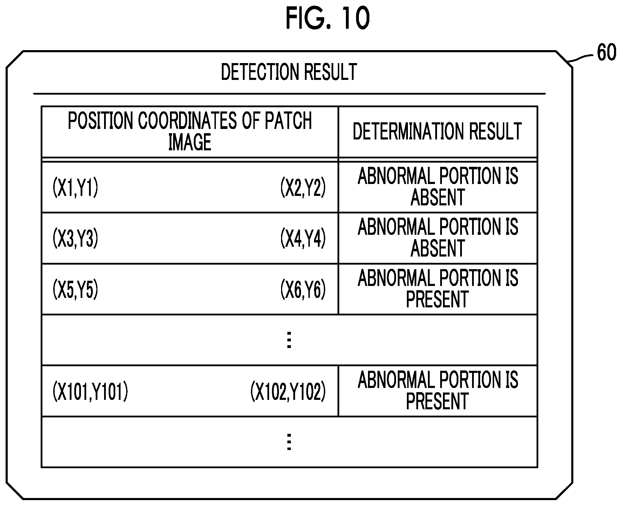

For example, as illustrated in Fig. 10, the detection result 60 is information in which the determination result 82 is registered for the coordinates of each position of the patch image 65. The position coordinates of the patch image 65 are, for example, the position coordinates of diagonal points of the patch image 65.

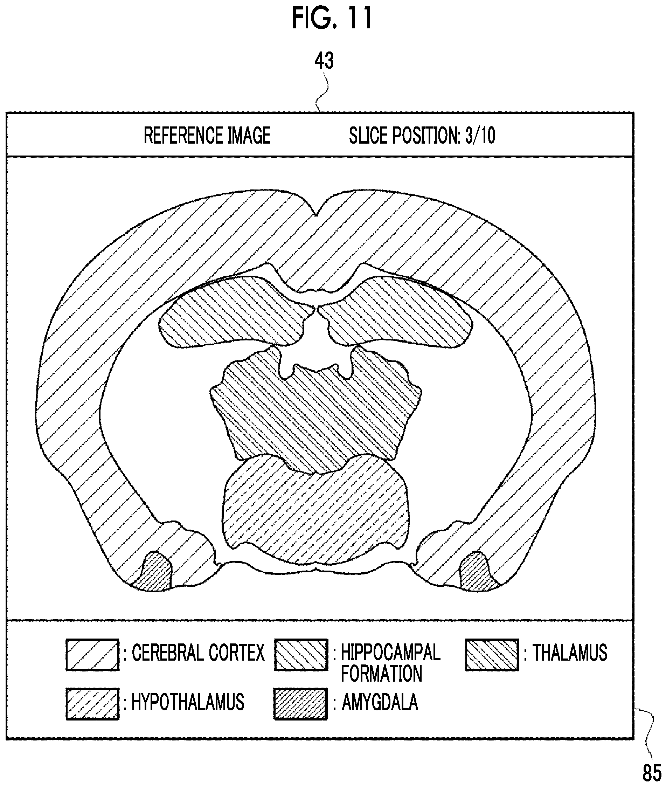

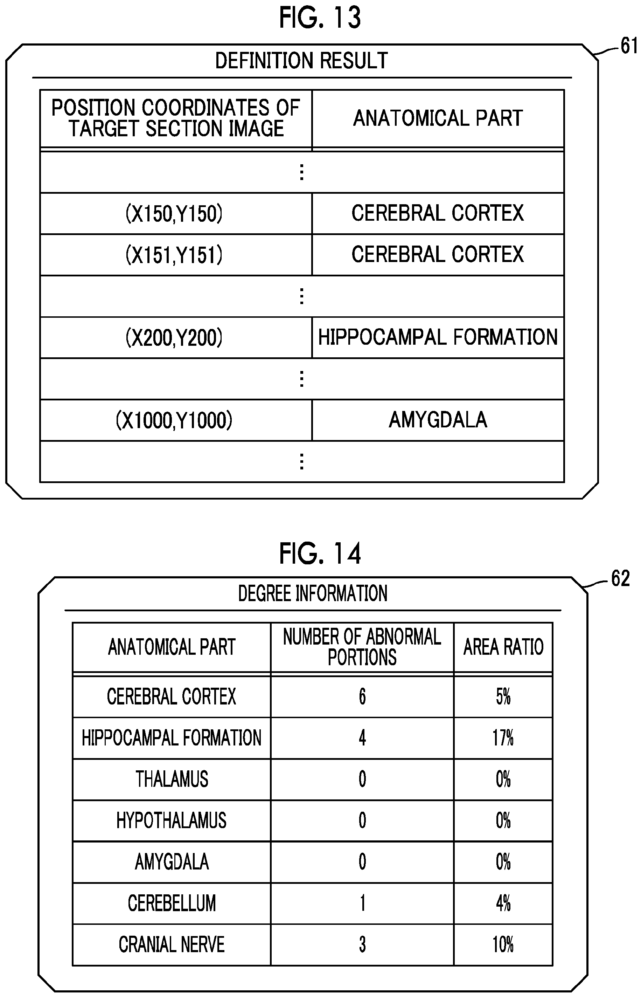

For example, as illustrated in Fig. 11, the reference image 43 is an image having positional information of a plurality of anatomical parts of the brain B. More specifically, the reference image 43 is an image in which a pixel value (for example, the cerebral cortex has a pixel value of 0, and the hippocampal formation has a pixel value of 1) indicating an anatomical part is registered in each corresponding pixel. The reference image 43 is called an atlas. In Fig. 11, as illustrated in a legend 85, the cerebral cortex, the hippocampal formation, the thalamus, the hypothalamus, and the amygdala are given as examples of the anatomical part. Examples of the anatomical part include the cerebellum and the cranial nerves in addition to these parts (see Fig. 14). The reference image 43 is prepared for each slice position of the coronal section of the brain section BS. The definition unit 52 registers the target section image 15T and the reference image 43 at the same slice position. Public data, such as Allen Brain Atlas provided by Allen Institute for Brain Science in Seattle, the United States, can be used as the reference image 43. In addition, the reference image 43 is not limited to the public data and may be an image originally created by a pharmaceutical facility.

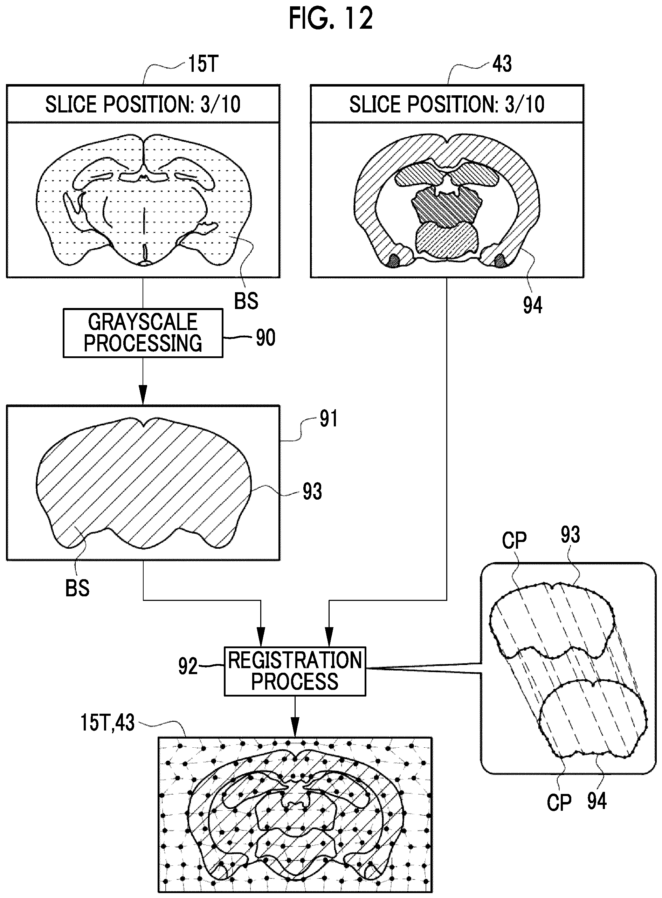

For example, as illustrated in Fig. 12, first, the definition unit 52 performs grayscale processing 90 on the target section image 15T to convert the target section image 15T into a grayscale image 91 in order to enhance a contour 93 of the brain section BS. Then, the definition unit 52 performs a registration process 92 of registering the grayscale image 91 with the reference image 43. In addition, the grayscale processing 90 may not be necessarily performed.

The registration process 92 is a non-rigid registration process and is performed, for example, by the following procedure. That is, a plurality of control points CP are set on the contour 93 of the brain section BS of the grayscale image 91. Then, each control point CP is assigned to the same position as that on a contour 94 of the brain section BS of the reference image 43. The definition unit 52 derives the amount of deformation of each pixel of the reference image 43 in a case in which the target section image 15T and the reference image 43 are registered with each other from the amount of movement of each control point CP of the contour 94 with respect to each control point CP of the contour 93 using, for example, thin plate spline conversion. The definition unit 52 deforms the reference image 43 with the derived amount of deformation and registers the target section image 15T with the reference image 43. In Fig. 12, the distortion of lattice points of the reference image 43 caused by the deformation of the target section image 15T for the registration is represented by thin lines. Further, for example, a non-rigid registration process using a B-spline curve or a non-rigid registration process considering brightness information of the target section image 15T may be performed instead of the non-rigid registration process using the thin plate spline conversion.

For example, as illustrated in Fig. 13, the definition result 61 is information in which the anatomical part is registered for the coordinates of each position of the target section image 15T.

For example, as illustrated in Fig. 14, the degree information 62 includes the number of abnormal portions in each of a plurality of anatomical parts and the area ratio of the abnormal portion in each of the plurality of anatomical parts. The number of abnormal portions and the area ratio of the abnormal portion are values derived for all of a plurality of section images 15 obtained from one subject S. The area ratio of the abnormal portion is the ratio of the number of pixels of the abnormal portion to the number of pixels of each anatomical part in all of the plurality of section images 15 obtained from one subject S. For example, in a case in which the number of pixels defined as the cerebral cortex by the definition unit 52 is 10,000 and the number of pixels in the region of interest 80 detected as the abnormal portion by the detection unit 51 is 500 in all of the plurality of section images 15 obtained from one subject S, the area ratio is (500/10000) × 100 = 5%.

For example, as illustrated in Fig. 15, the display control unit 54 performs control to display the drug discovery support screen 100A on the display 11. The display control unit 54 displays the drug discovery support screen 100A in a case where the operator inputs an instruction to display the section image 15 through the input device 12 and the instruction receiving unit 120 receives the display instruction. The drug discovery support screen 100A is provided with a pull-down menu 101 for selecting any one of the administration group 25 or the control group 27 and a pull-down menu 102 for selecting the subject ID of the subject S. Fig. 15 illustrates an example in which the administration group 25 is selected in the pull-down menu 101 and a subject ID "R001" is selected in the pull-down menu 102.

A plurality of section images 15 are displayed side by side on the drug discovery support screen 100A. The plurality of section images 15 are all of the section images 15 obtained from one subject S. Fig. 15 illustrates an example in which ten first section images 151 with image IDs "SP001" to "SP010" obtained from the subject S with the subject ID "R001" in the administration group 25 are displayed side by side.

An abnormality determination button 103 is provided in a lower portion of the drug discovery support screen 100A. In a case where the operator selects the abnormality determination button 103, the instruction receiving unit 120 receives an abnormality determination instruction. The abnormality determination instruction is an instruction for causing the detection unit 51, the definition unit 52, and the generation unit 53 to perform the detection of the abnormal portion from the section image 15 displayed on the drug discovery support screen 100A, the definition of the anatomical part in the section image 15 displayed on the drug discovery support screen 100A, and the generation of the degree information 62 for all of the section images 15 displayed on the drug discovery support screen 100A. That is, the section image 15 (here, the first section image 151 obtained from the subject S with the subject ID "R001" in the administration group 25) displayed on the drug discovery support screen 100A corresponds to the target section image 15T.

In a case where the abnormality determination button 103 is selected and the detection of the abnormal portion, the definition of the anatomical part, and the generation of the degree information 62 are performed, the display control unit 54 displays the drug discovery support screen 100B in a pop-up manner on the drug discovery support screen 100A as illustrated in Fig. 16 as an example. The degree information 62 is displayed on the drug discovery support screen 100B. The displayed drug discovery support screen 100B disappears in a case where an OK button 105 is selected.

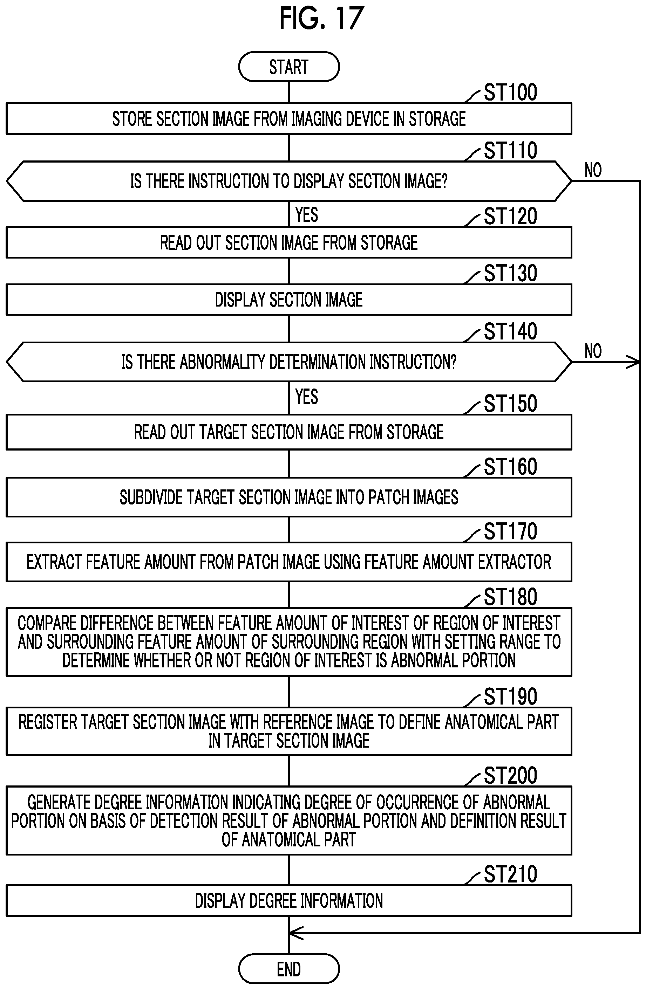

Next, the operation of the above-described configuration will be described with reference to a flowchart illustrated in Fig. 17 as an example. First, in a case where the operation program 40 is started in the drug discovery support apparatus 10, the CPU 32 of the drug discovery support apparatus 10 functions as the RW control unit 50, the detection unit 51, the definition unit 52, the generation unit 53, and the display control unit 54 as illustrated in Fig. 4.

First, the imaging device 19 captures the section image 15 obtained by imaging the brain section BS of the subject S. The section image 15 is output from the imaging device 19 to the drug discovery support apparatus 10. In the drug discovery support apparatus 10, the RW control unit 50 stores the section image 15 from the imaging device 19 in the storage 30 (Step ST100).

In a case where the operator inputs an instruction to display the section image 15 through the input device 12 (YES in Step ST110), the RW control unit 50 reads out the section image 15 designated by the display instruction from the storage 30 (Step ST120). The section image 15 is output from the RW control unit 50 to the display control unit 54. As illustrated in Fig. 15, the section image 15 is displayed on the display 11 through the drug discovery support screen 100A under the control of the display control unit 54 (Step ST130).

In a case where the operator selects the abnormality determination button 103 of the drug discovery support screen 100A to input the abnormality determination instruction (YES in Step ST140), the RW control unit 50 reads out the section image 15 displayed on the drug discovery support screen 100A at that time as the target section image 15T from the storage 30 to acquire the section image 15 (Step ST150). The target section image 15T is output from the RW control unit 50 to the detection unit 51 and the definition unit 52.

The feature amount extractor 41 and the setting range information 42 are read out from the storage 30 by the RW control unit 50 and are output to the detection unit 51. Further, the reference image 43 is read out from the storage 30 by the RW control unit 50 and is output to the definition unit 52.

As illustrated in Fig. 5, the detection unit 51 subdivides the target section image 15T into a plurality of patch images 65 (Step ST160). Then, as illustrated in Fig. 6, the detection unit 51 extracts the feature amount 68 from the patch image 65 using the feature amount extractor 41 (Step ST170).

As illustrated in Fig. 9, the detection unit 51 calculates the difference Δ between the feature amount of interest 68IN of the region of interest 80 and the surrounding feature amount 68S of the surrounding region 81. Then, the difference Δ and the setting range of the setting range information 42 are compared with each other, and the determination result 82 indicating whether or not the region of interest 80 is the abnormal portion is output (Step ST180). The detection result 60 illustrated in Fig. 10 is generated on the basis of the determination result 82. The detection result 60 is output from the detection unit 51 to the generation unit 53.

As illustrated in Fig. 12, the definition unit 52 registers the target section image 15T and the reference image 43 at the same slice position to define a plurality of anatomical parts of the brain B in the target section image 15T (Step ST190). Then, the definition result 61 illustrated in Fig. 13 is generated. The definition result 61 is output from the definition unit 52 to the generation unit 53.

The generation unit 53 generates the degree information 62 that is illustrated in Fig. 14 and that indicates the degree of occurrence of the abnormal portion for each of the plurality of anatomical parts on the basis of the detection result 60 and the definition result 61 (Step ST200). The degree information 62 is output from the generation unit 53 to the display control unit 54.

As illustrated in Fig. 16, the degree information 62 is displayed on the display 11 through the drug discovery support screen 100B under the control of the display control unit 54 (Step ST210). The operator evaluates the candidate substance 26 on the basis of the degree information 62.

As described above, the CPU 32 of the drug discovery support apparatus 10 comprises the RW control unit 50, the detection unit 51, the definition unit 52, and the display control unit 54. The RW control unit 50 reads out the target section image 15T obtained by imaging the brain B of the subject S from the storage 30 to acquire the target section image 15T. The detection unit 51 detects the abnormal portion in which an abnormality is estimated to occur from the target section image 15T. The definition unit 52 registers the target section image 15T with the reference image 43 having the positional information of the plurality of anatomical parts of the brain B to define the anatomical parts in the target section image 15T. The display control unit 54 displays the degree information 62 indicating the degree of occurrence of the abnormal portion in each of the plurality of anatomical parts on the drug discovery support screen 100B to output the degree information 62. Therefore, it is possible to easily ascertain how much the abnormality occurs in each of the anatomical parts of the brain B.

As a method for defining the anatomical part in the target section image 15T, a method is considered that applies a relatively simple image recognition technique such as pattern matching. However, in the image recognition technique, such as pattern matching, the accuracy of defining the anatomical part is relatively low. Therefore, in the technology of the present disclosure, the reference image 43 and the target section image 15T are registered with each other to define the anatomical part in the target section image 15T, thereby improving the accuracy of defining the anatomical part.

The detection unit 51 extracts the feature amount of interest 68IN of the region of interest 80 in the target section image 15T and determines whether or not the region of interest 80 is the abnormal portion on the basis of the difference Δ between the feature amount of interest 68IN and the surrounding feature amount 68S which is the reference feature amount. Therefore, it is possible to easily determine whether or not the region of interest 80 is the abnormal portion.

The abnormal portion is a portion, such as a tumor, inflammation, a cyst, or an infiltrate, that has a shape and a texture different from those of the surroundings. Therefore, in a case in which the reference feature amount is the surrounding feature amount 68S extracted from the surrounding region 81 of the region of interest 80, it is possible to increase the accuracy of detecting the abnormal portion.

The degree information 62 includes the number of abnormal portions in each of a plurality of anatomical parts and the area ratio of the abnormal portions in each of the plurality of anatomical parts. Therefore, it is possible to more accurately ascertain how much the abnormality occurs in each of the anatomical parts.

The organ image is the section image 15 obtained by imaging the brain section BS. In the section image 15, the brain section BS is stained with a coloring agent as a whole, which makes it difficult to know the structural features of the brain B. Therefore, in a case where a relatively simple image recognition technique, such as pattern matching, is applied, there is a concern that the accuracy of defining the anatomical part will be very low. For this reason, the anatomical part is defined by registration with the reference image 43, which makes it possible to further exhibit the effect of increasing the accuracy of defining the anatomical part.

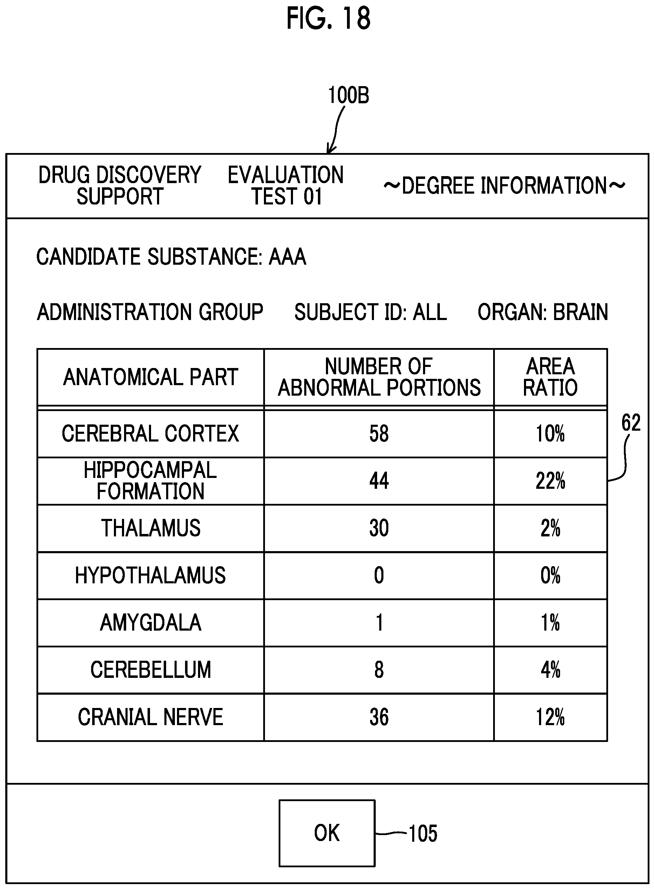

Further, an option for designating all of the subjects S constituting the administration group 25 or the control group 27 may be provided in the pull-down menu 102 of the drug discovery support screen 100A. Then, as in a drug discovery support screen 100B illustrated in Fig. 18 as an example, for example, the degree information 62 generated by using the section images 15 of all of the subjects S in the administration group 25 as the target section images 15T may be displayed. This enables the operator to know how much the abnormality considered to be caused by the candidate substance 26 occurs in each of the anatomical parts in the administration group 25. In addition, in a case where the control group 27 is selected in the pull-down menu 102, the operator can know how much the abnormality considered to be caused not by the candidate substance 26, but by, for example, the attribute and/or the breeding environment of the subject S occurs in each of the anatomical parts in the control group 27.

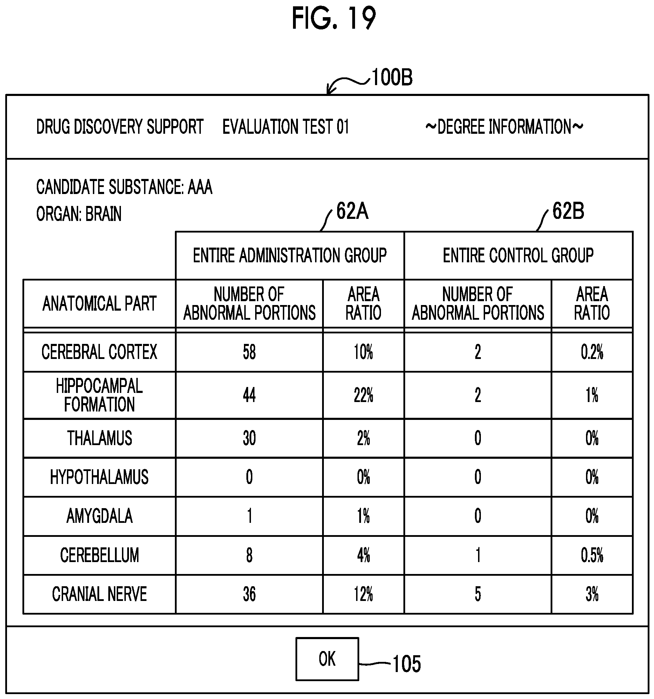

In addition, an option for designating both the administration group 25 and the control group 27 at the same time may be provided in the pull-down menu 101 of the drug discovery support screen 100A. Then, as in a drug discovery support screen 100B illustrated in Fig. 19 as an example, degree information 62A and degree information 62B may be displayed side by side. The degree information 62A is degree information 62 generated by using the section images 15 of all of the subjects S constituting the administration group 25 as the target section images 15T. The degree information 62B is degree information 62 generated by using the section images 15 of all of the subjects S constituting the control group 27 as the target section images 15T. This enables the operator to easily ascertain how much the abnormality considered to be caused by the candidate substance 26 and the abnormality considered to be caused by the attribute and/or the breeding environment of the subject S occur in each of the anatomical parts.

### [Second Embodiment]

In the first embodiment, the surrounding feature amount 68S extracted from the surrounding region 81 of the region of interest 80 is given as an example of the reference feature amount. However, the present disclosure is not limited thereto.

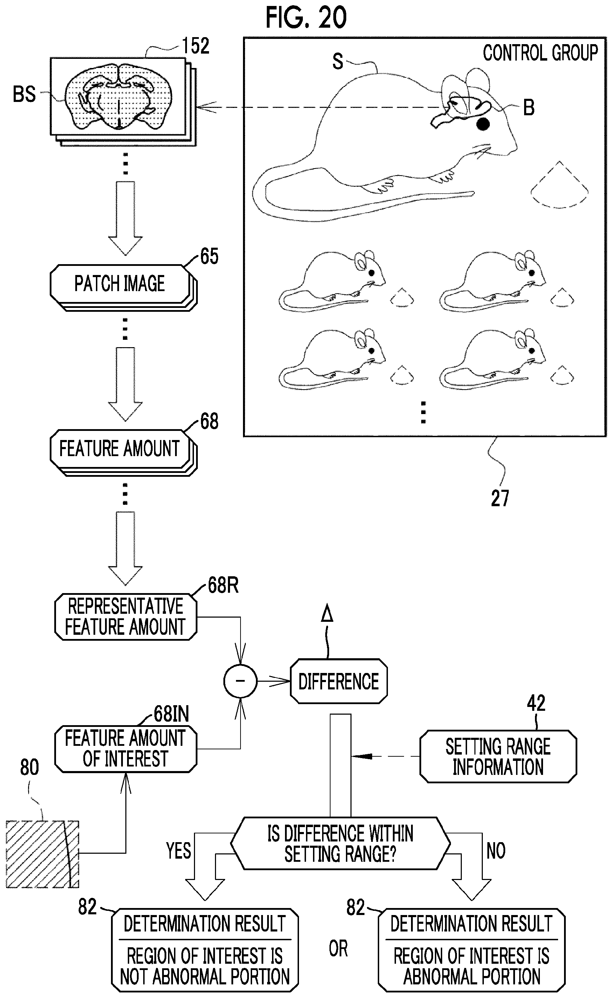

For example, as illustrated in Fig. 20, in a second embodiment, a representative feature amount 68R is used as the reference feature amount. The representative feature amount 68R is a representative value, for example, an average value or the like of a plurality of feature amounts 68 extracted from a plurality of patch images 65 obtained by subdividing a second section image 152 obtained from the subject S constituting the control group 27. Since the process of the detection unit 51 is the same as the process illustrated in Fig. 9 in the first embodiment except that the surrounding feature amount 68S is the representative feature amount 68R, a description thereof will not be repeated.

As described above, the control group 27 is a collection of the subjects S to which the candidate substance 26 for a drug has not been administered. In the subjects S to which the candidate substance 26 has not been administered, of course, the abnormality caused by the candidate substance 26 do not occur. Therefore, the abnormal portion can be detected with high accuracy by using, as the reference feature amount, the representative feature amount 68R extracted from the second section image 152 of the subject S to which the candidate substance 26 has not been administered.

In addition, the representative feature amount 68R of the feature amounts 68 extracted from the second section images 152 of a plurality of subjects S to which the candidate substance has not been administered in the evaluation test of the candidate substance for a drug developed in the past may be used as the reference feature amount.

### [3 _1-th Embodiment]

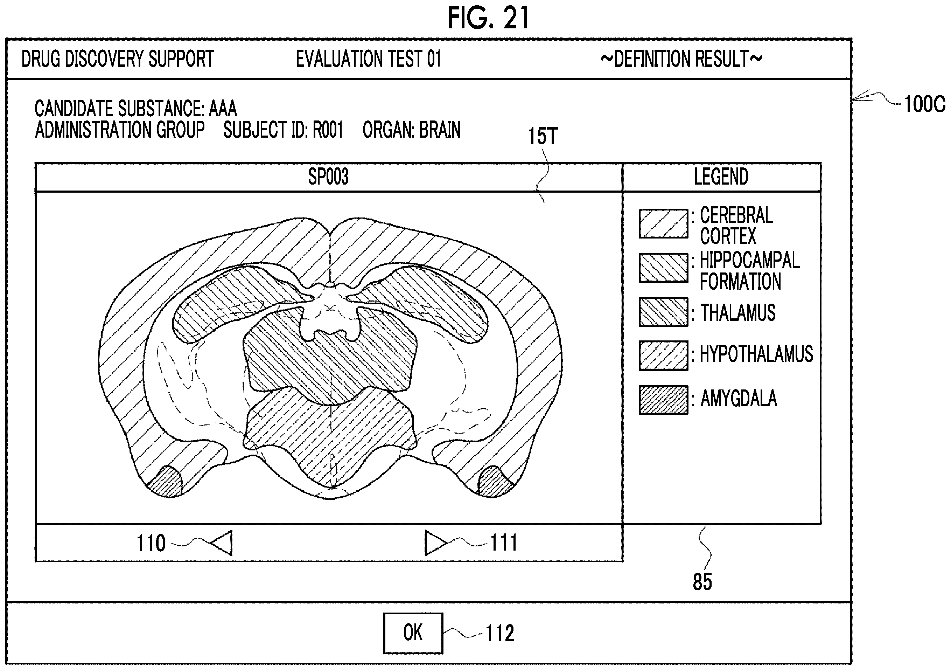

For example, as illustrated in Fig. 21, in a 3_1-th embodiment, the display control unit 54 performs control to display a drug discovery support screen 100C on the display 11. The display control unit 54 displays the drug discovery support screen 100C before the generation unit 53 generates the degree information 62 (before the display of the drug discovery support screen 100B) or after the generation unit 53 generates the degree information 62 (after the display of the drug discovery support screen 100B).

One target section image 15T is displayed on the drug discovery support screen 100C. The anatomical parts defined by the definition unit 52 are displayed to be superimposed on the target section image 15T. The legend 85 of the anatomical parts is displayed on the right side of the target section image 15T. In addition, a back button 110 and a forward button 111 for switching the display of the target section image 15T are provided below the target section image 15T. The displayed drug discovery support screen 100C disappears in a case where an OK button 112 is selected.

As described above, in the 3_1-th embodiment, the display control unit 54 performs control to display the defined anatomical parts on the display 11 to be superimposed on the target section image 15T. Therefore, the operator can check the validity of the defined anatomical parts.

### [3_2-th Embodiment]

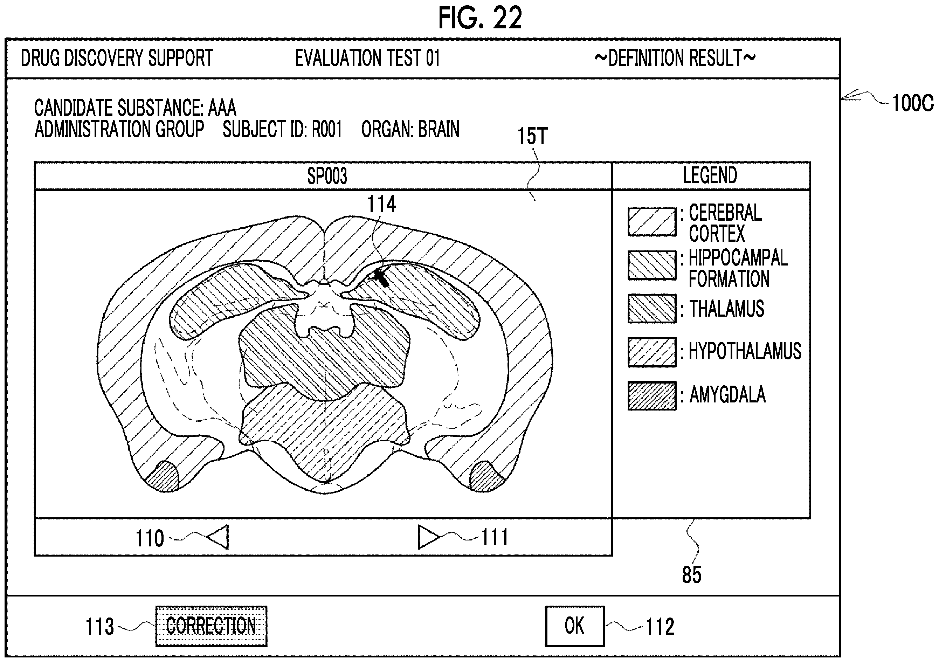

For example, as illustrated in Fig. 22, in a 3_2-th embodiment, the operator can correct the defined anatomical part on the drug discovery support screen 100C. Specifically, a correction button 113 is provided in a lower portion of the drug discovery support screen 100C. While the correction button 113 is selected, for example, the contour of the anatomical part on the target section image 15T can be corrected by using a cursor 114 of a mouse. This correction of the anatomical part is confirmed in a case where the OK button 112 is selected.

For example, as illustrated in Fig. 23, in the 3_2-th embodiment, the instruction receiving unit 120 receives an instruction 121 to correct the anatomical part from the operator through the input device 12. The correction instruction 121 includes the position coordinates of pixels of the contour of the anatomical part corrected by the operator using the cursor 114. The instruction receiving unit 120 outputs the correction instruction 121 to the definition unit 52. The definition unit 52 corrects the definition result 61 according to the correction instruction 121 to obtain a corrected definition result 61C. The definition unit 52 outputs the corrected definition result 61C to the generation unit 53.

The generation unit 53 generates the degree information 62 on the basis of the corrected definition result 61C. In the aspect in which the drug discovery support screen 100C is displayed before the generation unit 53 generates the degree information 62 (before the display of the drug discovery support screen 100B), the degree information 62 displayed on the drug discovery support screen 100B is generated on the basis of the corrected definition result 61C from the beginning. On the other hand, in the aspect in which the drug discovery support screen 100C is displayed after the generation unit 53 generates the degree information 62 (after the display of the drug discovery support screen 100B), the degree information 62 displayed on the drug discovery support screen 100B is switched from the degree information 62 generated on the basis of the definition result 61 before the correction to the degree information 62 generated on the basis of the corrected definition result 61C.

As described above, in the 3_2-th embodiment, the instruction receiving unit 120 receives the instruction 121 to correct the defined anatomical part. Therefore, the operator can manually correct the anatomical part defined by the definition unit 52.

In addition, the display control unit 54 outputs the degree information 62 generated on the basis of the anatomical part corrected according to the correction instruction 121. Therefore, the degree information 62 based on the anatomical part manually corrected by the operator can be provided for viewing by the operator.

In each of the above-described embodiments, the slide specimen 18 on which only the brain section BS is placed is given as an example. However, the present disclosure is not limited thereto. A slide specimen in which not only the brain section BS but also tissue sections of a plurality of types of organs, such as a heart section and a bone marrow section, are placed on one slide glass 16 may be handled. In this case, an identification unit that identifies the tissue section of each organ is constructed in the CPU 32. The identification unit identifies the tissue section of each organ. Then, the subsequent process, such as the detection of an abnormal portion, is performed. This configuration makes it possible to respond to a slide specimen in which tissue sections of a plurality of types of organs are placed. For the slide specimen, the slide specimen in which the tissue sections of a plurality of types of organs are placed is more common than the slide specimen 18 in which the tissue section of one organ is placed as in the first embodiment. Therefore, it is possible to perform drug discovery support that is more suitable for general operations.

The encoder unit 71 of the autoencoder 70 is given as an example of the feature amount extractor 41. However, the present disclosure is not limited thereto. An encoder unit of a convolutional neural network which has been trained to output a determination result of whether or not an abnormality is present in a certain class, for example, the brain section BS included in the patch image 65 in response to the input of the patch image 65 may be used as the feature amount extractor 41. In addition, another machine learning model, such as a support vector machine, may be used as the feature amount extractor 41.

In the tissue section included in the patch image 65L for learning, a region that is likely to be erroneously detected as an abnormality may be masked and then used for learning. Examples of the region that is likely to be erroneously detected as an abnormality include a blood vessel region in the brain section BS and dust stuck in a case where the slide specimen 18 is created.

The feature amount 68 is not limited to the feature amount extracted by the feature amount extractor 41. The feature amount 60 may be, for example, the average value, maximum value, minimum value, mode value, or variance of the pixel values of the patch image 65.

The anatomical parts are not limited to the cerebral cortex, the hippocampal formation, the amygdala, and the like which are given as examples. For example, the hippocampal formation may be subdivided into the dentate gyrus, the hippocampus, the subiculum, the entorhinal cortex, and the like, or the hippocampus may be subdivided into each region of CA1, CA2, and CA3. A plurality of types of reference images 43 having different classifications of anatomical parts may be stored and used for the purpose.

The organ is not limited to the brain B or the like that is given as an example. The organ may be a stomach, a lung, a small intestine, a large intestine, or the like. In addition, the subject S is not limited to the rat. The subject S may be a mouse, a guinea pig, a sand mouse, a hamster, a ferret, a rabbit, a dog, a cat, a monkey, or the like.

The organ image is not limited to the section image 15 that is given as an example. The organ image may be a tomographic image by computed tomography (CT), a tomographic image by magnetic resonance imaging (MRI), a tomographic image by positron emission tomography (PET), a tomographic image by single photon emission computed tomography (SPECT), or the like.

The output form of the degree information 62 is not limited to the drug discovery support screen 100B given as an example in the first embodiment and includes printout on paper or file output by an e-mail attachment or the like.

The drug discovery support apparatus 10 may be a personal computer that is installed in a pharmaceutical facility as illustrated in Fig. 1 or may be a server computer that is installed in a data center independent of the pharmaceutical facility.

In a case where the drug discovery support apparatus 10 is configured by the server computer, the section image 15 is transmitted from the personal computer installed in each pharmaceutical facility to the server computer via a network such as the Internet. The server computer delivers the drug discovery support screen 100A or the like to the personal computer in the form of screen data for web delivery created by a markup language such as Extensible Markup Language (XML). The personal computer reproduces the drug discovery support screen 100A or the like to be displayed on the web browser on the basis of the screen data and displays the drug discovery support screen 100A or the like on the display. Further, instead of XML, another data description language, such as JavaScript (registered trademark) Object Notation (JSON), may be used.

The drug discovery support apparatus 10 according to the technology of the present disclosure can be widely used throughout all stages of drug development from the setting of a drug discovery target, which is the initial stage, to a clinical trial which is the final stage.

The hardware configuration of the computer constituting the drug discovery support apparatus 10 according to the technology of the present disclosure can be modified in various ways. For example, the drug discovery support apparatus 10 may be configured by a plurality of computers that are separated as hardware for the purpose of improving processing capability and reliability. For example, the functions of the detection unit 51 and the functions of the definition unit 52 may be distributed to two computers. In this case, the drug discovery support apparatus 10 is configured by the two computers.

As described above, the hardware configuration of the computer of the drug discovery support apparatus 10 can be changed as appropriate depending on required performance such as processing capacity, safety, and reliability. Further, not only the hardware but also an application program, such as the operation program 40, can be duplicated or distributed and stored in a plurality of storages for the purpose of ensuring safety and reliability.

In each of the above-described embodiments, for example, the following various processors can be used as a hardware structure of processing units executing various processes, such as the RW control unit 50, the detection unit 51, the definition unit 52, the generation unit 53, the display control unit 54, and the instruction receiving unit 120. The various processors include, for example, the CPU 32 which is a general-purpose processor executing software (operation program 40) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to execute a specific process.

One processing unit may be configured by one of the various processors or by a combination of two or more processors of the same type or different types (for example, a combination of a plurality of FPGAs and/or a combination of a CPU and an FPGA). Further, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system on chip (SoC). As described above, the various processing units are configured by using one or more of the above various processors as the hardware structure.

In addition, more specifically, an electric circuit (circuitry) in which circuit elements, such as semiconductor elements, are combined can be used as the hardware structure of these various processors.

In the technology of the present disclosure, the above-described various embodiments and/or various modification examples may be combined with each other as appropriate. In addition, it goes without saying that the present disclosure is not limited to each of the above-described embodiments, and various configurations can be adopted without departing from the gist of the present disclosure. Furthermore, the technology of the present disclosure extends to a storage medium that non-temporarily stores a program, in addition to the program.

The above descriptions and illustrations are detailed descriptions of portions related to the technology of the present disclosure and are merely examples of the technology of the present disclosure. For example, the above description of the configurations, functions, operations, and effects is the description of examples of the configurations, functions, operations, and effects of portions related to the technology of the present disclosure. Therefore, unnecessary portions may be deleted or new elements may be added or replaced in the above descriptions and illustrations without departing from the gist of the technology of the present disclosure. In addition, in the above descriptions and illustrations, the description of, for example, common technical knowledge that does not need to be particularly described to enable the implementation of the technology of the present disclosure is omitted in order to avoid confusion and facilitate the understanding of portions related to the technology of the present disclosure.

In the present specification, "A and/or B" is synonymous with "at least one of A or B". That is, "A and/or B" means only A, only B, or a combination of A and B. Further, in the present specification, the same concept as "A and/or B" is applied to a case where the connection of three or more matters is expressed by "and/or".

All of the publications, the patent applications, and the technical standards described in the present specification are incorporated by reference herein to the same extent as each individual document, each patent application, and each technical standard are specifically and individually stated to be incorporated by reference.

## Claims

1. An information processing apparatus comprising:
a processor configured to:
acquire an organ image obtained by imaging an organ of a subject;
detect an abnormal portion, in which an abnormality is estimated to occur, from the organ image;
register the organ image with a reference image having positional information of a plurality of anatomical parts of the organ to define the anatomical parts in the organ image; and
output degree information indicating a degree of occurrence of the abnormal portion in each of the plurality of anatomical parts.

2. The information processing apparatus according to claim 1, wherein the processor is configured to:
extract a feature amount of a region of interest in the organ image; and
determine whether or not the region of interest is the abnormal portion on the basis of a difference between the feature amount extracted from the region of interest and a reference feature amount.

3. The information processing apparatus according to claim 2,
wherein the reference feature amount is a feature amount extracted from a region around the region of interest.

4. The information processing apparatus according to claim 2,
wherein the organ image is an image used in an evaluation test for evaluating a candidate substance for a drug, and
the reference feature amount is a feature amount extracted from an organ image of a subject to which the candidate substance has not been administered.

5. The information processing apparatus according to any one of claims 1 to 4, wherein the processor is configured to:
perform control to display the defined anatomical part on a display to be superimposed on the organ image.

6. The information processing apparatus according to claim 5, wherein the processor is configured to:
receive an instruction to correct the defined anatomical part.

7. The information processing apparatus according to claim 6, wherein the processor is configured to:
output the degree information generated on the basis of the anatomical part corrected according to the correction instruction.

8. The information processing apparatus according to any one of claims 1 to 7,
wherein the degree information includes the number of the abnormal portions in each of the plurality of anatomical parts and an area ratio of the abnormal portion in each of the plurality of anatomical parts.

9. The information processing apparatus according to any one of claims 1 to 8,
wherein the organ image is a section image obtained by imaging a tissue section of the organ.

10. A method for operating an information processing apparatus, the method comprising:
acquiring an organ image obtained by imaging an organ of a subject;
detecting an abnormal portion, in which an abnormality is estimated to occur, from the organ image;
registering the organ image with a reference image having positional information of a plurality of anatomical parts of the organ to define the anatomical parts in the organ image; and
outputting degree information indicating a degree of occurrence of the abnormal portion in each of the plurality of anatomical parts.

11. A program for operating an information processing apparatus, the program causing a computer to execute a process comprising:
acquiring an organ image obtained by imaging an organ of a subject;
detecting an abnormal portion, in which an abnormality is estimated to occur, from the organ image;
registering the organ image with a reference image having positional information of a plurality of anatomical parts of the organ to define the anatomical parts in the organ image; and
outputting degree information indicating a degree of occurrence of the abnormal portion in each of the plurality of anatomical parts.

## Amended claims

### Amended claims under Art. 19.1 PCT

1. (Amended) An information processing apparatus comprising:
a processor configured to:
acquire an organ image obtained by imaging an organ of a subject, the organ image being an image used in an evaluation test for evaluating a candidate substance for a drug;
detect an abnormal portion, in which an abnormality is estimated to occur, from the organ image;
register the organ image with a reference image having positional information of a plurality of anatomical parts of the organ to define the anatomical parts in the organ image; and
output degree information indicating a degree of occurrence of the abnormal portion in each of the plurality of anatomical parts, wherein
in detecting the abnormal portion, all of the subjects constituting an administration group to which the candidate substance is administered and/or all of the subjects constituting a control group to which the candidate substance is not administered can be targeted, and
as the degree information, it is possible to output degree information generated for all of the subjects constituting the administration group, and/or degree information generated for all of the subjects constituting the control group.

2. The information processing apparatus according to claim 1, wherein the processor is configured to:
extract a feature amount of a region of interest in the organ image; and
determine whether or not the region of interest is the abnormal portion on the basis of a difference between the feature amount extracted from the region of interest and a reference feature amount.

3. The information processing apparatus according to claim 2,
wherein the reference feature amount is a feature amount extracted from a region around the region of interest.

4. The information processing apparatus according to claim 2,
wherein the organ image is an image used in an evaluation test for evaluating a candidate substance for a drug, and
the reference feature amount is a feature amount extracted from an organ image of a subject to which the candidate substance has not been administered.

5. The information processing apparatus according to any one of claims 1 to 4, wherein the processor is configured to:
perform control to display the defined anatomical part on a display to be superimposed on the organ image.

6. The information processing apparatus according to claim 5, wherein the processor is configured to:
receive an instruction to correct the defined anatomical part.

7. The information processing apparatus according to claim 6, wherein the processor is configured to:
output the degree information generated on the basis of the anatomical part corrected according to the correction instruction.

8. The information processing apparatus according to any one of claims 1 to 7,
wherein the degree information includes the number of the abnormal portions in each of the plurality of anatomical parts and an area ratio of the abnormal portion in each of the plurality of anatomical parts.

9. The information processing apparatus according to any one of claims 1 to 8,
wherein the organ image is a section image obtained by imaging a tissue section of the organ.

10. (Amended) A method for operating an information processing apparatus, the method comprising:
acquiring an organ image obtained by imaging an organ of a subject, the organ image being an image used in an evaluation test for evaluating a candidate substance for a drug;
detecting an abnormal portion, in which an abnormality is estimated to occur, from the organ image;
registering the organ image with a reference image having positional information of a plurality of anatomical parts of the organ to define the anatomical parts in the organ image; and
outputting degree information indicating a degree of occurrence of the abnormal portion in each of the plurality of anatomical parts, wherein
in detecting the abnormal portion, all of the subjects constituting an administration group to which the candidate substance is administered and/or all of the subjects constituting a control group to which the candidate substance is not administered can be targeted, and
as the degree information, it is possible to output degree information generated for all of the subjects constituting the administration group, and/or degree information generated for all of the subjects constituting the control group.

11. (Amended) A program for operating an information processing apparatus, the program causing a computer to execute a process comprising:
acquiring an organ image obtained by imaging an organ of a subject, the organ image being an image used in an evaluation test for evaluating a candidate substance for a drug;
detecting an abnormal portion, in which an abnormality is estimated to occur, from the organ image;
registering the organ image with a reference image having positional information of a plurality of anatomical parts of the organ to define the anatomical parts in the organ image; and
outputting degree information indicating a degree of occurrence of the abnormal portion in each of the plurality of anatomical parts, wherein
in detecting the abnormal portion, all of the subjects constituting an administration group to which the candidate substance is administered and/or all of the subjects constituting a control group to which the candidate substance is not administered can be targeted, and
as the degree information, it is possible to output degree information generated for all of the subjects constituting the administration group, and/or degree information generated for all of the subjects constituting the control group.

12. (Added) The information processing apparatus according to any one of claims 2 to 4, wherein the processor is configured to:
gradually change the size of the region of interest to extract the feature amount and determine whether or not the region of interest is the abnormal portion.
